# EUROPEAN PATENT APPLICATION

(11) **EP 4 681 525 A1**
(43) Date of publication of application: **21.01.2026**
(21) Application number: 25190269.8
(22) Date of filing: 17.07.2025
(51) Int. Cl.: A01D 45/02, A01D 41/127

(54) **ROW UNIT CONTROL SYSTEM FOR AN AGRICULTURAL HARVESTER**

(30) Priority: 18.07.2024 US 202418776714
(71) Applicant: CNH Industrial America LLC, New Holland, PA 17557 (US)
(72) Inventor: D'Amicantonio, Matthew, New Holland, 17557 (US)
(74) Representative: CNH Industrial IP Department

(57) **Abstract**

A row unit control system for an agricultural harvester includes a controller having a memory and a processor. The controller is configured to identify an absence of crops along a respective path of each row unit of a set of row units of the agricultural harvester. In addition, the controller is configured to terminate operation of at least one component of the row unit in response to identifying the absence of the crops along the respective path of the row unit.

## Description

### BACKGROUND

The present disclosure relates generally to a row unit control system for an agricultural harvester.

An agricultural harvester may be used to harvest agricultural crops, such as barley, beans, beets, carrots, corn, cotton, flax, oats, potatoes, rye, soybeans, wheat, or other plant crops. The agricultural harvester may include a header, which may be configured to efficiently harvest certain types of crops. For example, a corn header may be configured to efficiently harvest corn. The corn header may include row units that include components configured to separate ears of corn from stalks as the agricultural harvester travels through a field. Augers carry the ears of corn toward a processing system of the agricultural harvester, and the stalks are deposited on the field.

### BRIEF DESCRIPTION

In certain embodiments, a row unit control system for an agricultural harvester includes a controller having a memory and a processor. The controller is configured to identify an absence of crops along a respective path of each row unit of a set of row units of the agricultural harvester. In addition, the controller is configured to terminate operation of at least one component of the row unit in response to identifying the absence of the crops along the respective path of the row unit.

### BRIEF DESCRIPTION OF THE DRAWINGS

These and other features, aspects, and advantages of the present disclosure will become better understood when the following detailed description is read with reference to the accompanying drawings in which like characters represent like parts throughout the drawings, wherein:
FIG. 1 is a side view of an embodiment of an agricultural harvester having a corn header;
FIG. 2 is a perspective view of an embodiment of a corn header that may be employed within the agricultural harvester of FIG. 1;
FIG. 3 is a perspective front view of a portion of the corn header of FIG. 2;
FIG. 4 is a perspective bottom view of a portion of the corn header of FIG. 2;
FIG. 5 is a schematic diagram of an embodiment of a row unit control system that may be employed within the agricultural harvester of FIG. 1; and
FIG. 6 is a flow diagram of an embodiment of a method for controlling row units of an agricultural harvester.

### DETAILED DESCRIPTION

One or more specific embodiments of the present disclosure will be described below. In an effort to provide a concise description of these embodiments, all features of an actual implementation may not be described in the specification. It should be appreciated that in the development of any such actual implementation, as in any engineering or design project, numerous implementation-specific decisions must be made to achieve the developers' specific goals, such as compliance with system-related and business-related constraints, which may vary from one implementation to another. Moreover, it should be appreciated that such a development effort might be complex and time consuming, but would nevertheless be a routine undertaking of design, fabrication, and manufacture for those of ordinary skill having the benefit of this disclosure.

When introducing elements of various embodiments of the present disclosure, the articles "a," "an," "the," and "said" are intended to mean that there are one or more of the elements. The terms "comprising," "including," and "having" are intended to be inclusive and mean that there may be additional elements other than the listed elements. Any examples of operating parameters and/or environmental conditions are not exclusive of other parameters/conditions of the disclosed embodiments.

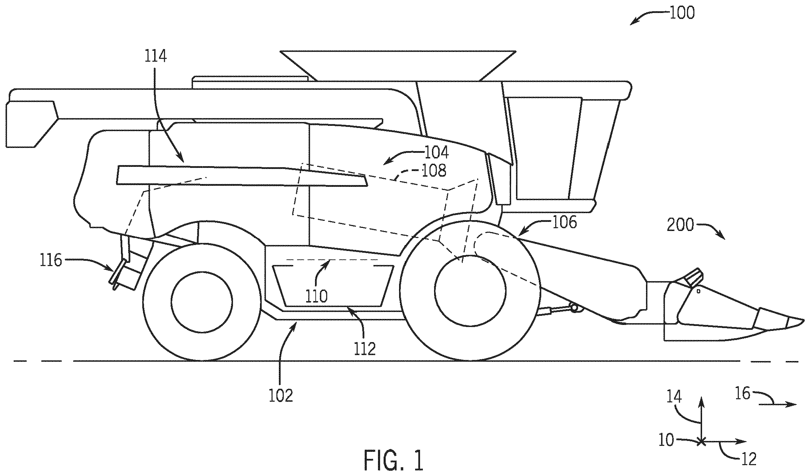

FIG. 1 is a side view of an embodiment of an agricultural harvester 100 having a corn header 200 (e.g., agricultural header). The agricultural harvester 100 includes a chassis 102 configured to support the corn header 200 and an agricultural crop processing system 104. As described in greater detail below, the corn header 200 is configured to separate ears of corn from stalks and to transport the ears of corn toward an inlet 106 of the agricultural crop processing system 104 for further processing of the ears of corn. The agricultural crop processing system 104 receives the ears of corn from the corn header 200 and separates desired crop material from crop residue. For example, the agricultural crop processing system 104 may include a thresher 108 having a cylindrical threshing rotor that transports the ears of corn in a helical flow path through the agricultural harvester 100. In addition to transporting the ears of corn, the thresher 108 may separate certain desired crop material (e.g., corn kernels) from the crop residue, such as husks and cobs, and enable the desired crop material to flow into a cleaning system 110 located beneath the thresher 108. The cleaning system 110 may remove debris from the desired crop material and transport the desired crop material to a storage compartment 112 within the agricultural harvester 100. The crop residue may be transported from the thresher 108 to a crop residue handling system 114, which may remove the crop residue from the agricultural harvester 100 via a crop residue spreading system 116 positioned at the aft end of the agricultural harvester 100.

As discussed in detail below, the corn header 200 includes multiple row units configured to separate ears of corn from stalks, thereby leaving bare stalks engaged with the soil. The ears of corn are directed toward the inlet 106. The bare stalks that remain engaged with the soil may be collectively referred to as stubble. To facilitate discussion, the agricultural harvester 100 and/or its components (e.g., the corn header 200) may be described with reference to a lateral axis or direction 10, a longitudinal axis or direction 12, and a vertical axis or direction 14. The agricultural harvester 100 and/or its components (e.g., the corn header 200) may also be described with reference to a direction of travel 16.

In certain embodiments, the agricultural harvester 100 includes a row unit control system configured to control the row units of the corn header 200. The row unit control system includes a controller having a memory and a processor. The controller is configured to identify absence of crops along a respective path of each row unit. The controller is also configured to terminate operation of at least one component of the row unit in response to identifying the absence of the crops along the respective path of the row unit. For example, as the agricultural harvester 100 moves through a field along the direction of travel 16, at least one row unit of the corn header 200 may encounter a region of the field that has already been harvested (e.g., by the agricultural harvester during a previous pass through the field). In response to identifying the absence of the crops along the respective path(s) of the row unit(s), the controller of the row unit control system may selectively terminate operation of component(s) of the row unit(s). Terminating operation of the component(s) of the row unit(s) may reduce the energy used by the corn header during the harvesting operation, thereby reducing the fuel used by the agricultural harvester. As a result, the efficiency of the harvesting operation may be enhanced.

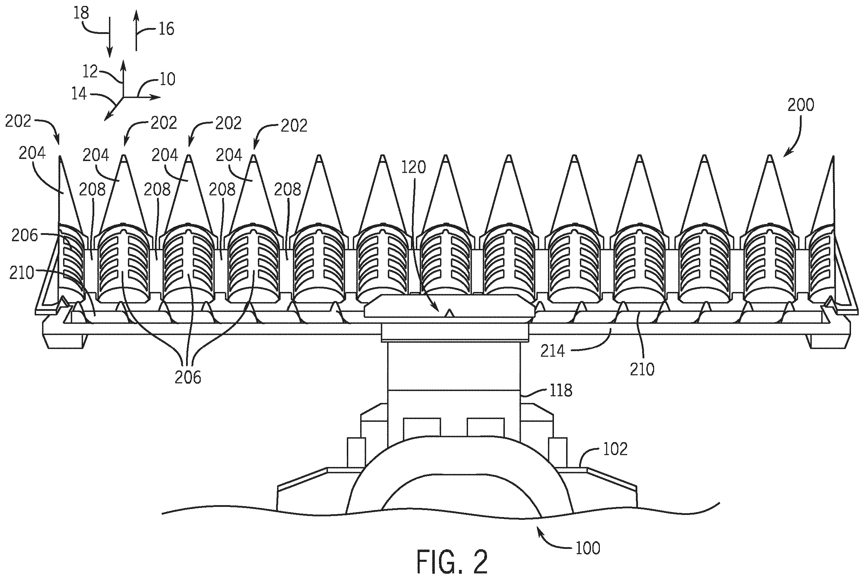

FIG. 2 is a perspective view of an embodiment of a corn header 200 that may be employed within the agricultural harvester 100 of FIG. 1. In the illustrated embodiment, the corn header 200 includes multiple crop engagement assemblies 202 distributed along the lateral axis 10 of the corn header 200. Each crop engagement assembly 202 includes a divider 204 and a hood 206. The dividers 204 are configured to divide rows of crops (e.g., corn), and the hoods 206 are configured to block crop material from entering internal components of the corn header 200. The crop engagement assemblies 202 may be evenly spaced along the lateral axis 10 of the corn header 200. As the corn header 200 moves along the direction of travel 16, the dividers 204 direct crop material from each row of crops into a respective row unit 208. In addition, the hoods 206 facilitate alignment of the crop material with the row units 208. Accordingly, the hoods 206 direct the crop material through the row units 208.

In the illustrated embodiment, the corn header 200 includes a pair of augers 210, and the augers 210, the row units 208, and the crop engagement assemblies 202 are supported by a frame 214 of the corn header 200. The frame 214 of the corn header 200 is coupled to a feeder house 118 of the agricultural harvester 100. Furthermore, the row units 208 are configured to separate ears of corn from stalks, thereby leaving bare stalks engaged with the soil (e.g., stubble). The ears of corn may be directed to one of the pair of augers 210, which are configured to convey the ears of corn inwardly along the lateral axis 10 of the corn header 200 to a feeder 120 at the lateral center of the corn header 200. As illustrated, the augers 210 extend along a substantial portion of the width of the corn header 200 (e.g., along the lateral axis 10 of the corn header 200). The augers 210 may be driven by a driving mechanism (e.g., electric motor, hydraulic motor, etc.). As the agricultural harvester 100 moves through the field, the dividers 204 direct the rows of crops/crop material into the row units 208. The row units 208 engage the crop material within the field and separate the ears of corn from the stalks, and the augers 210 transport the ears of corn to the feeder 120, which directs the ears of corn toward the inlet of the agricultural crop processing system. Each row unit 208 includes feed rollers which pull the stalk of each crop downwardly through the row unit during harvesting. As the stalks of the crops are pulled through the feed rollers, the ears are separated from the stalks and are conveyed toward the augers 210.

In addition to pulling the stalks of the crops downwardly, the feed rollers drive the stalks rearwardly (e.g., along a rearward direction 18 opposite the direction of travel 16) relative to the frame 214 of the corn header 200. Due to the movement of the agricultural harvester 100, the frame 214 of the corn header 200 is driven to move forwardly along the direction of travel 16. The difference between the forward speed of the corn header frame 214 relative to the field and the rearward speed of the stalks relative to the corn header frame 214 may be referred to as the conveyance speed. A conveyance speed of about zero may enhance the efficiency of the harvesting operation (e.g., by reducing the loads/forces applied to the ears of corn) and/or may enhance subsequent agricultural operation(s) (e.g., by enhancing the engagement of the bare stalks with the soil).

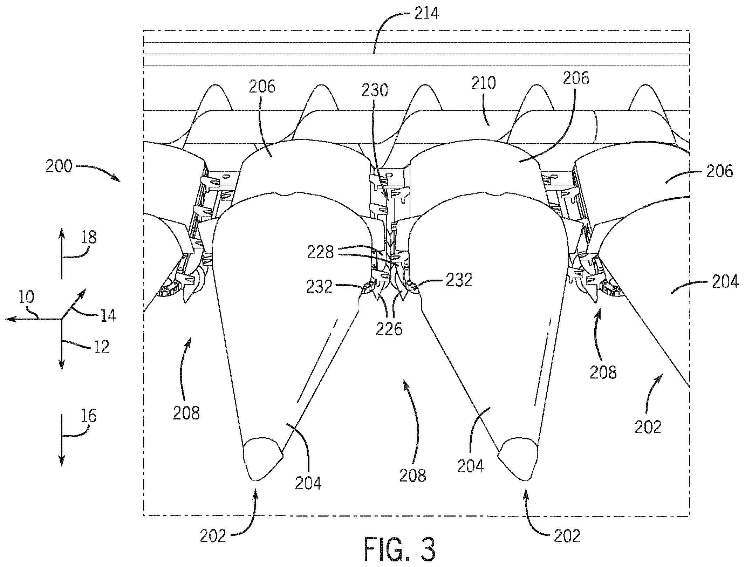

FIG. 3 is a perspective front view of a portion of the corn header 200 of FIG. 2. As previously discussed, the corn header 200 includes multiple dividers 204 that direct the crops into the row units 208. Each row unit 208 is configured to separate the ear of corn from the stalk, carry the ear of corn toward the respective auger 210, and direct the stalk to the field. As illustrated, each row unit 208 includes a pair of feed rollers 226 configured to grip the stalk and to rotate in opposite directions, thereby driving the stalk rearwardly (e.g., along the rearward direction 18) and toward the field (e.g., vertically downward, below the corn header 200). Each row unit 208 also includes a pair of deck plates 228 positioned over the pair of feed rollers 226. Each deck plate 228 extends along the longitudinal axis 12 of the corn header 200, and the pair of deck plates 228 are separated from one another along the lateral axis 10 of the corn header 200 to define a gap 230. Furthermore, each row unit 208 includes a pair of chains 232 (e.g., with lugs) that are configured to drive or push the ear of corn along the pair of deck plates 228 toward the respective auger 210. The pair of deck plates 228 are spaced apart so that the gap 230 is sized to enable the stalk to fall through the gap 230 and to block the ear of corn from falling through the gap 230. In some embodiments, the pair of deck plates are adjustable and may be driven (e.g., via an actuator) toward and away from one another along the lateral axis 10 to change a size (e.g., width) of the gap. Each hood 206 is positioned rearward of each divider 204 and between adjacent row units 208 to cover various components, such as the actuator that drives the pair of deck plates 228, linkage(s), and so forth. While each row unit 208 includes feed rollers 226, deck plates 228, and chains 232 in the illustrated embodiment, in other embodiments, at least one row unit may include other/additional suitable component(s) configured to facilitate separating the ear of corn from the stalk, directing the ear of corn to the respective auger, and directing the stalk toward the surface of the agricultural field.

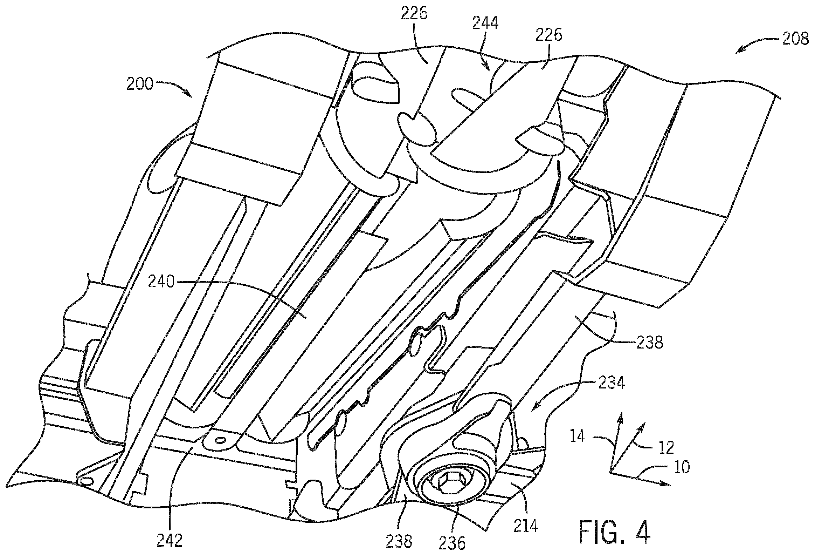

FIG. 4 is a perspective bottom view of a portion of the corn header 200 of FIG. 2. In the illustrated embodiment, each row unit 208 of the corn header 200 includes a chopper 234 positioned below the feed rollers 226 with respect to the vertical axis 14 of the corn header 200. Each chopper is configured to chop the stalk into multiple pieces as the stalk moves through the feed rollers 226. Accordingly, a portion of the stalk is chopped into residue, and the remainder of the stalk remains engaged with the soil (e.g., stubble).

In the illustrated embodiment, the chopper 234 includes a hub 236 rotatably coupled to the frame 214 of the corn header 200. In addition, the chopper 234 includes blades 238 coupled to the hub 236 and extending radially outwardly from the hub 236. In the illustrated embodiment, the chopper 234 has two blades 238. However, in other embodiments, the chopper may include more or fewer blades (e.g., 1, 3, 4, or more). As discussed in detail below, the chopper 234 may be driven to rotate by a driven shaft, and a clutch may be coupled to the driven shaft to selectively initiate and terminate rotation of the chopper.

In the illustrated embodiment, each row unit 208 of the corn header 200 includes a counter knife 240 (e.g., a counter blade), which may enhance the effectiveness of the chopper 234 in cutting the stalk. For example, the counter knife 240 may support the stalk being moved by the feed rollers 226 and provide resistance to enable the chopper 234 to more effectively cut the stalk. The chopper 234 may cut the stalk against the counter knife 240, such that each of the chopper 234 and the counter knife 240 imparts a force against the stalk to cut the stalk. As such, the counter knife 240 may enhance operation of the chopper 234. The counter knife 240 may be coupled to a support 242 of the frame 214 and may extend (e.g., generally along the longitudinal axis 12) toward a front end 244 (e.g., an upstream end, a tip) of the corn header 200. A portion of the counter knife 240 may overlap with the blades 238 of the chopper 234 during rotation of the chopper 234.

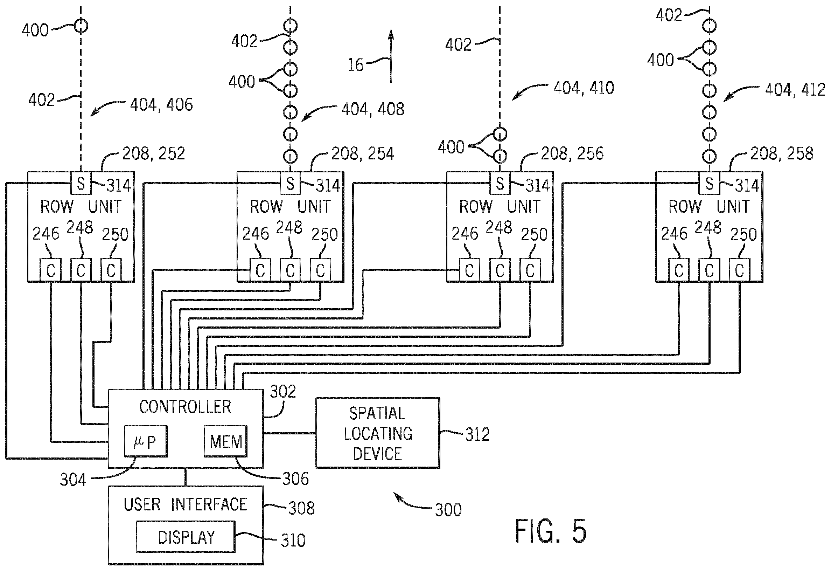

FIG. 5 is a schematic diagram of an embodiment of a row unit control system 300 that may be employed within the agricultural harvester of FIG. 1. As previously discussed, in certain embodiments, each row unit 208 includes a pair of feed rollers configured to grip the stalk and to rotate in opposite directions, thereby driving the stalk rearwardly and toward the field. **In** addition, in certain embodiments, each row unit 208 includes a pair of chains configured to drive or push the ear of corn along the pair of deck plates toward the respective auger. Furthermore, in certain embodiments, each row unit 208 includes a chopper configured to chop the stalk into multiple pieces as the stalk passes through the feed rollers.

In certain embodiments, the feed rollers, the chains, and the choppers are driven to rotate by a driven shaft that is driven to rotate by an engine of the agricultural harvester. For example, the driven shaft may be coupled to a transmission that is driven by the engine of the agricultural harvester, or the driven shaft may be coupled to a hydraulic motor that is powered by a hydraulic pump coupled to the engine. In the illustrated embodiment, each row unit 208 includes a first clutch 246 (e.g., clutch) coupled to the driven shaft and to the chopper of the row unit. The first clutch 246 may be selectively engaged and disengaged to selectively initiate and terminate rotation of the chopper. Furthermore, in the illustrated embodiment, each row unit 208 includes a second clutch 248 coupled to the driven shaft and to the pair of chains of the row unit. The second clutch 248 may be selectively engaged and disengaged to selectively initiate and terminate movement of the pair of chains. **In** addition, in the illustrated embodiment, each row unit 208 includes a third clutch 250 coupled to the driven shaft and to the pair of feed rollers of the row unit. The third clutch 250 may be selectively engaged and disengaged to selectively initiate and terminate rotation of the feed rollers.

While each of the chopper, the pair of feed rollers, and the pair of chains is coupled to a respective clutch in the illustrated embodiment, in other embodiments, at least one of the chopper, the pair of feed rollers, or the pair of chains may not be coupled to a clutch. For example, in certain embodiments, the second and third clutches may be omitted, such that operation of the pair of feed rollers and the pair of chains is not controllable independently of the driven shaft (e.g., the pair of feed rollers and the pair of chains are in operation while the driven shaft is rotating). Furthermore, in certain embodiments, a single clutch may control multiple components of the row unit. In such embodiments, multiple components of the row unit may be coupled to the single clutch. For example, in certain embodiments, the pair of feed rollers and the pair of chains may be coupled to a single clutch, such that the single clutch may be controlled to selectively initiate and terminate operation of the pair of feed rollers and the pair of chains.

In the illustrated embodiment, the row unit control system 300 includes a controller 302 communicatively coupled to the first clutch 246, the second clutch 248, and the third clutch 250 of each row unit 208. In certain embodiments, the controller 302 is an electronic controller having electrical circuitry configured to control the first clutch 246, the second clutch 248, and the third clutch 250 of each row unit 208. In the illustrated embodiment, the controller 302 includes a processor, such as the illustrated microprocessor 304, and a memory device 306. The controller 302 may also include one or more storage devices and/or other suitable components. The processor 304 may be used to execute software, such as software for controlling the first clutch 246, the second clutch 248, and the third clutch 250 of each row unit 208, and so forth. Moreover, the processor 304 may include multiple microprocessors, one or more "general-purpose" microprocessors, one or more special-purpose microprocessors, and/or one or more application specific integrated circuits (ASICs), or some combination thereof. For example, the processor 304 may include one or more reduced instruction set (RISC) processors.

The memory device 306 may include a volatile memory, such as random access memory (RAM), and/or a nonvolatile memory, such as read-only memory (ROM). The memory device 306 may store a variety of information and may be used for various purposes. For example, the memory device 306 may store processor-executable instructions (e.g., firmware or software) for the processor 304 to execute, such as instructions for controlling the first clutch 246, the second clutch 248, and the third clutch 250 of each row unit 208, and so forth. The storage device(s) (e.g., nonvolatile storage) may include ROM, flash memory, a hard drive, or any other suitable optical, magnetic, or solid-state storage medium, or a combination thereof. The storage device(s) may store data, instructions (e.g., software or firmware for controlling the first clutch 246, the second clutch 248, and the third clutch 250 of each row unit 208, etc.), and any other suitable data.

In the illustrated embodiment, the corn ear monitoring system 300 includes a user interface 308 communicatively coupled to the controller 302. The user interface 308 is configured to receive input from an operator and to provide information to the operator. The user interface 308 may include any suitable input device(s) for receiving input, such as a keyboard, a mouse, button(s), switch(es), knob(s), other suitable input device(s), or a combination thereof. In addition, the user interface 308 may include any suitable output device(s) for presenting information to the operator, such as speaker(s), indicator light(s), other suitable output device(s), or a combination thereof. In the illustrated embodiment, the user interface 308 includes a display 310 configured to present visual information to the operator. In certain embodiments, the display 310 may include a touchscreen interface configured to receive input from the operator.

In the illustrated embodiment, the controller 302 is configured to identify an absence of crops 400 along a respective path 402 of each row unit 208 of the header of the agricultural harvester. As illustrated, the path 402 of each row unit 208 follows a row 404 of the crops 400, such that the row unit 208 engages the crops 400 within the row 404 as the agricultural harvester moves through the field along the direction of travel 16. In addition, the controller 302 is configured to terminate operation of at least one component of the row unit (e.g., the chopper, the pair of feed rollers, the pair of chains, or a combination thereof) in response to identifying the absence of the crops 400 along the respective path 402 of the row unit 208.

For example, a first row unit 252 of the header is aligned with a respective first row 406 of the crops 400. As illustrated, crops are absent in front of the first row unit 252 relative to the direction of travel 16. Accordingly, the controller 302 may identify the absence of the crops along the respective path 402 of the first row unit 252. As such, the controller 302 may terminate operation of at least one component of the first row unit 252. Furthermore, a second row unit 254 of the header is aligned with a respective second row 408 of the crops 400. As illustrated, crops are present in front of the second row unit 254 relative to the direction of travel 16. Accordingly, the controller 302 may not identify the absence of the crops along the respective path 402 of the second row unit 254. As such, the controller 302 may not terminate operation of any component of the second row unit 254. In addition, a third row unit 256 of the header is aligned with a respective third row 410 of the crops 400. As illustrated, a small number of crops are present in front of the third row unit 256 relative to the direction of travel 16. Accordingly, the controller 302 may not initially identify the absence of the crops along the respective path 402 of the third row unit 256. As such, the controller 302 may not initially terminate operation of any component of the third row unit 256. However, forward of the small number of crops 400 relative to the direction of travel 16, crops are absent. Accordingly, as the third row unit 256 reaches the region of crop absence, the controller 302 may identify the absence of the crops along the respective path 402 of the third row unit 256. As such, the controller 302 may terminate operation of at least one component of the third row unit 256. Furthermore, a fourth row unit 258 of the header is aligned with a respective fourth row 412 of crops 400. As illustrated, crops are present in front of the fourth row unit 258 relative to the direction of travel 16. Accordingly, the controller 302 may not identify the absence of the crops along the respective path 402 of the fourth row unit 258. As such, the controller 302 may not terminate operation of any component of the fourth row unit 258. While the corn header includes four row units in the illustrated embodiment, in other embodiments, the corn header may include more or fewer row units.

In certain embodiments, the controller 302 is configured to identify the absence of the crops along the respective path of each row unit based on a crop coverage map of the field and a location of the agricultural harvester within the field. The crop coverage map includes one or more regions where the crop is present within the field and/or one or more regions where the crop is absent from the field. For example, the crop coverage map may include at least one region where the crops are absent because the agricultural harvester previously harvested the crops within the region. Accordingly, in certain embodiments, the controller 302 is configured to update the coverage map as the agricultural harvester traverses the field and the row units harvest the crop from the field. Furthermore, the crop coverage map may include one or more regions where the crops are absent because no crop was planted in the region(s) (e.g., because the region(s) include soil unsuitable for planting, seed was not deposited within the region(s) because the seeder/planter was not operating within the region(s), etc.).

In the illustrated embodiment, the row unit control system 300 includes a spatial locating device 312 (e.g., global positioning system (GPS) receiver, inertial measurement unit (IMU), etc.) communicatively coupled to the controller 302. The spatial locating device 312 is configured to output a position signal to the controller 302 indicative of the location of the agricultural harvester within the field. The controller 302 is configured to determine the location of the agricultural harvester within the field based on the position signal, and the controller 302 is configured to identify the absence of the crops along the respective path of each row unit 208 based on the crop coverage map and the location of the agricultural harvester. For example, the spatial locating device 312 may be positioned at a reference location on the agricultural harvester, and the controller 302 may store a set of positions of the row units relative to the reference location. Accordingly, the controller 302 may determine the location of each row unit within the field based on the position of the reference location within the field and the position of the row unit relative to the reference location. The controller 302 may then identify the absence of the crops along the respective path of the row unit based on the crop coverage map and the location of the row unit within the field.

In the illustrated embodiment, the row unit control system 300 includes sensors 314 communicatively coupled to the controller 302. Each sensor 314 is configured to output a sensor signal indicative of the absence of the crops along the respective path 402 of the respective row unit 208, and the controller 302 is configured to identify the absence of the crops along the respective path 402 of the respective row unit 208 based on feedback from the sensor 314. In the illustrated embodiment, a single sensor 314 is configured to monitor crops along the respective path 402 of each row unit 208. However, in other embodiments, multiple sensors may be configured to monitor crops along the respective path of at least one row unit.

Each sensor 314 may include any suitable type(s) of sensing device(s) configured to monitor presence and/or absence of crops along the respective path 402. For example, in certain embodiments, the sensor 314 may include a radio detection and ranging (RADAR) sensing device configured to output a radio frequency (RF) signal and detect a return signal. Furthermore, in certain embodiments, the sensor 314 may include a camera configured to receive an image of the row of crops. In addition, in certain embodiments, the sensor 314 may include an infrared transceiver configured to output an infrared (IR) signal and detect a return signal. Furthermore, in certain embodiments, the sensor 314 may include an ultrasonic sensor configured to output an ultrasonic signal and detect a return signal. The sensor may also include any other suitable sensing device(s) (e.g., alone or in combination with any of the sensing devices disclosed above) configured to monitor presence or absence of crops along the respective path.

Furthermore, in certain embodiments, the sensor 314 may include a force or torque sensor configured to monitor the load on at least one component of the row unit 208 (e.g., the chopper, the pair of chains, the pair of feed rollers, or a combination thereof). In such embodiments, the controller 302 may be configured to identify the absence of the crops along the respective path 402 in response to determining the load on the component(s) is below a threshold value. Furthermore, in certain embodiments, the controller 302 may be configured to identify the absence of the crops along the respective path 402 in response to determining the load on the component(s) is below a threshold value for a threshold duration (e.g., 1 second, 2 seconds, 3 seconds, 4 seconds, 5 seconds, etc.).

While each row 404 of crops 400 is monitored by a respective sensor 314 in the illustrated embodiment, in other embodiments, one sensor may be configured to monitor multiple rows of crops. For example, in certain embodiments, a single sensor (e.g., camera, RADAR sensing device, etc.) may be configured to monitor all of the rows of crops engaged by the row units of the header. Furthermore, while identifying the absence of the crops along the respective path of each row unit based on feedback from sensor(s) and based on a coverage map of the field and the location of the agricultural harvester is disclosed above, in certain embodiments, the controller may be configured to identify the absence of the crops along the respective path of each row unit based on feedback from the sensor(s) alone (e.g., without using the crop coverage map), or the controller may be configured to identify the absence of the crops along the respective path of each row unit based on the coverage map and the location of the agricultural harvester alone (e.g., without feedback from the sensor(s)).

In certain embodiments, the controller 302 is configured to selectively initiate and terminate operation of the component(s) of each row unit 208 by controlling the respective clutches. For example, in response to identifying the absence of the crops along the respective path 402 of the row unit 208, the controller 302 may terminate operation of the chopper by disengaging the first clutch 246 coupled to the chopper, terminate operation of the pair of chains by disengaging the second clutch 248 coupled to the pair of chains, terminate operation of the pair of feed rollers by disengaging the third clutch 250 coupled to the pair of feed rollers, or a combination thereof. Furthermore, in response to identifying presence of crops along the respective path 402 of the row unit 208, the controller may initiate operation of the chopper by engaging the first clutch 246 coupled to the chopper, initiate operation of the pair of chains by engaging the second clutch 248 coupled to the pair of chains, initiate operation of the pair of feed rollers by engaging the third clutch 250 coupled to the pair of feed rollers, or a combination thereof. In embodiments in which the row unit has one or two clutches, the controller may control the one or two clutches to selectively initiate and terminate operation of the respective component(s).

In certain embodiments, the chopper, the pair of chains, the pair of feed rollers, or a combination thereof, may be driven by one or more motors (e.g., electric motor(s), hydraulic motor(s), pneumatic motor(s), etc.). In such embodiments, the controller may control the motor(s) to control operation of the component(s) of the row unit. For example, in certain embodiments, the chopper of each row unit may be driven to rotate by a respective motor. In such embodiments, the controller may be communicatively coupled to the motor, the controller may engage the motor to initiate operation of the chopper, and the controller may disengage the motor to terminate operation of the chopper. Furthermore, in certain embodiments, the pair of chains and the pair of feed rollers of each row unit may be driven to rotate by a respective single motor. In such embodiments, the controller may be communicatively coupled to the single motor, the controller may engage the single motor to initiate operation of the pair of feed rollers and the pair of chains, and the controller may disengage the single motor to terminate operation of the pair of feed rollers and the pair of chains.

As previously discussed, the controller 302 is configured to terminate operation of the component(s) in response to identifying the absence of the crops along the respective path 402 of the row unit 208. Furthermore, in certain embodiments, the controller 302 is configured to initiate operation of the component(s) in response to identifying the presence of the crops along the respective path 402 of the row unit 208. In certain embodiments, the controller 302 is configured to determine a location of the absence of the crops in front of the row unit 208 along the respective path 402, and the controller 302 is configured to terminate operation of the component(s) a threshold distance before the location. For example, in certain embodiments, the controller 302 may terminate operation of the chopper at the threshold distance before the location to enable rotation of the blade(s) of the chopper to stop at or shortly after the location. The threshold location may be selected to enable the inertia of the rotating blade(s) to continue to chop the stalk(s) without the chopper being driven to rotate. Because operation of the chopper is terminated before the row unit reaches the location of the absence of the crops, energy usage may be further reduced (e.g., as compared to terminating operation of the chopper at the location). In addition, in certain embodiments, the controller 302 is configured to determine a location of the presence of crops in front of the row unit 302 along the respective path 402 (e.g., while the crops are absent immediately in front of the row unit along the respective path). In such embodiments, the controller 302 is configured to initiate operation of the component(s) a threshold distance before the location. For example, in certain embodiments, the controller 302 may initiate operation of the chopper at the threshold distance before the location to enable the blade(s) of the chopper to reach a target operational speed at the location. The threshold distance may be selected based on the angular acceleration of the blade(s) after rotation of the chopper is initiated.

In certain embodiments, the controller 302 is configured to terminate operation of the component(s) of each row unit 208 in response to transitioning the header from a working position to a non-working position (e.g., transport position, storage position, etc.). For example, in certain embodiments, a sensor may be coupled to the controller and configured to output a signal indicative of the position of the header. In such embodiments, the controller may terminate operation of the component(s) of each row unit in response to feedback from the sensor indicating that the header is in the non-working position. Furthermore, in certain embodiments, the controller may control actuator(s) to move the header from the working position to the non-working position. In such embodiments, the controller may concurrently terminate operation of the component(s) of each row unit and control the actuator(s) to move the header from the working position to the non-working position.

Furthermore, in certain embodiments, the controller 302 may be configured to control the user interface 308 to present information to the operator regarding the operation of the component(s) of the row units 208. For example, the controller 302 may instruct the display 310 of the user interface 308 to present a visual representation indicating whether each controllable component of each row unit 208 is in operation. Furthermore, in certain embodiments, the controller 302 may receive input from the user interface 308 indicative of instructions to selectively initiate or terminate operation of at least one component of at least one row unit (e.g., to override automatic control of the component(s)).

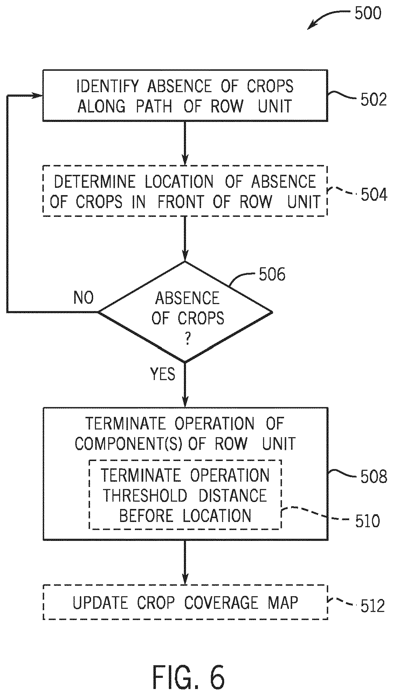

FIG. 6 is a flow diagram of an embodiment of a method 500 for controlling row units of an agricultural harvester. The method 500 may be performed by the controller disclosed above with reference to FIG. 5, by one or more other suitable controllers, or a combination thereof. Furthermore, the steps of the method 500 may be performed in the order disclosed below or in any other suitable order. In addition, in certain embodiments, one or more steps of the method 500 may be omitted, and/or the method may include one or more additional steps.

The method 500 for controlling row units of an agricultural harvester includes identifying an absence of crops along a respective path of each row unit, as represented by block 502. As previously discussed, in certain embodiments, identifying the absence of the crops along the respective path of the row unit is based on a crop coverage map of a field and a location of the agricultural harvester within the field. The crop coverage map of the field may include a region of crops harvested by the agricultural harvester. Furthermore, in certain embodiments, identifying the absence of the crops along the respective path of the row unit is based on feedback from a sensor configured to output a sensor signal indicative of the absence of the crops along the respective path. In certain embodiments, a location of the absence of the crops in front of the row unit along the respective path is determined, as represented by block 504.

As represented by block 506, a determination is made regarding whether absence of the crops along the respective path of the row unit is identified. In response to identifying the crops are not absent (e.g., present) along the respective path of the row unit, the method 500 returns to block 502. Furthermore, in response to identifying the crops are absent along the respective path of the row unit, the method 500 proceeds to block 508. As represented by block 508, operation of at least one component of the row unit is terminated. For example, operation of a chopper may be terminated by disengaging a first clutch coupled to the chopper, operation of a pair of chains may be terminated by disengaging a second clutch coupled to the pair of chains, operation of a pair of feed rollers may be terminated by disengaging a third clutch coupled to the pair of feed rollers, or a combination thereof. In embodiments in which the location of the absence of the crops in front of the row unit is determined, terminating operation of the component(s) of the row unit may include terminating operation of the component(s) a threshold distance before the location, as represented by block 510. For example, in certain embodiments, operation of the chopper may be terminated at the threshold distance before the location to enable rotation of the blade(s) of the chopper to stop at or shortly after the location, thereby further reducing energy usage (e.g., as compared to terminating operation of the chopper at the location). Furthermore, in embodiments in which identifying the absence of the crops along the respective path of the row unit is based on the crop coverage map of the field and the location of the agricultural harvester within the field, the coverage map may be updated as the agricultural harvester traverses the field, as represented by block 512.

## Claims

1. A row unit control system (300) for an agricultural harvester (100), comprising:
a controller (302) comprising a memory (306) and a processor (304), wherein the controller (302) is configured to:
identify an absence of crops (400) along a respective path (402) of each row unit of a plurality of row units (208, 252, 254, 256, 258) of the agricultural harvester (100); and
terminate operation of at least one component of the row unit (208, 252, 254, 256, 258) in response to identifying the absence of the crops (400) along the respective path (402) of the row unit.

2. The row unit control system (300) of claim 1, wherein the at least one component comprises a chopper (234).

3. The row unit control system (300) of claim 2, wherein the controller (302) is configured to terminate operation of the chopper (234) by disengaging a clutch (246, 248, 250) coupled to the chopper (234).

4. The row unit control system (300) according to any of the previous claims, wherein the controller (302) is configured to identify the absence of the crops (400) along the respective path (402) of the row unit (208, 252, 254, 256, 258) based on a crop coverage map of a field and a location of the agricultural harvester (100) within the field.

5. The row unit control system (300) of claim 4, wherein the crop coverage map includes a region of crops harvested by the agricultural harvester (100), and the controller (302) is configured to update the crop coverage map as the agricultural harvester (100) traverses the field.

6. The row unit control system (300) according to any of the claims 1 to 3, wherein the controller (302) is configured to identify the absence of the crops (400) along the respective path (402) of the row unit (208, 252, 254, 256, 258) based on feedback from a sensor (314).

7. The row unit control system (300) according to any of the previous claims, wherein the controller (302) is configured to:
determine a location of the absence of the crops (400) in front of the row unit (208, 252, 254, 256, 258) along the respective path (402); and
terminate operation of the at least one component of the row unit (208, 252, 254, 256, 258) a threshold distance before the location.

8. An agricultural harvester (100), comprising:
a plurality of row units (208, 252, 254, 256, 258), wherein each row unit of the plurality of row units is configured to receive crops (400) as the row unit moves along a respective path (402); and
a row unit control system (300) according to any of the previous claims.

9. A method for controlling a plurality of row units (208, 252, 254, 256, 258) of an agricultural harvester (100), comprising:
identifying, via a controller (302) comprising a memory (306) and a processor (304), an absence of crops (400) along a respective path (402) of each row unit (208, 252, 254, 256, 258) of the plurality of row units; and
terminating, via the controller (302), operation of at least one component of the row unit (208, 252, 254, 256, 258) in response to identifying the absence of the crops (400) along the respective path (402) of the row unit.

10. The method of claim 159 wherein the at least one component comprises a chopper (234).

11. The method of claim 10, wherein terminating operation of the chopper comprises disengaging, via the controller (302), a clutch (246, 248, 250) coupled to the chopper (234).

12. The method according to any of the claims 9 to 11, wherein identifying the absence of the crops (400) along the respective path (402) of the row unit (208, 252, 254, 256, 258) is based on a crop coverage map of a field and a location of the agricultural harvester (100) within the field.

13. The method of claim 12, comprising updating, via the controller (302), the crop coverage map as the agricultural harvester (100) traverses the field, wherein the crop coverage map includes a region of crops harvested by the agricultural harvester (100).

14. The method according to any of the claims 9 to 13, comprising determining, via the controller (302), a location of the absence of the crops (400) in front of the row unit (208, 252, 254, 256, 258) along the respective path (402), wherein terminating operation of the at least one component of the row unit (208, 252, 254, 256, 258) comprises terminating operation of the at least one component of the row unit a threshold distance before the location.
